# EUROPÄISCHE PATENTSCHRIFT

(11) **EP 0 692 985 B1**
(45) Veröffentlichungstag und Bekanntmachung des Hinweises auf die Patenterteilung: **11.08.1999**
(21) Anmeldenummer: 94913532.1
(22) Anmeldetag: 05.04.1994
(51) Int. Cl.: A61L 25/00

(54) **FILMBILDENDE WUNDGELE MIT DESINFIZIERENDER WIRKUNG**
DISINFECTANT FILM-FORMING WOUND-COVERING GEL
GEL FILMOGENE DE RECOUVREMENT DE PLAIES A EFFET DESINFECTANT

(30) Priorität: 05.04.1993 DE 4311131
(43) Veröffentlichungstag der Anmeldung: 24.01.1996
(73) Patentinhaber: Ritter, Günter, Dr., 32257 Bünde (DE)
(72) Erfinder: Ritter, Günter, Dr., 32257 Bünde (DE)
(74) Vertreter: Tesch, Rudolf, Dr.
(86) Internationale Anmeldenummer: EP9401055
(87) Internationale Veröffentlichungsnummer: WO9422504

(56) Entgegenhaltungen:
- US-A- 3 608 070

## Beschreibung

Die Erfindung betrifft filmbildende Wundgele mit desinfisierender Wirkung zur Förderung der Wundheilung bei Reptilien.

Hautwunden benötigen einen Schutz vor Staub und Bakterien. Besonders bei der Behandlung von Tieren sollte dieser leicht auftragbar und nicht ohne weiteres durch das Tier entfernbar sein. Andererseits sollte dieser die wundheilung nicht behindern, nicht mit der Wunde verkleben und vom Pfleger ohne größeren Aufwand entfernbar sein.

Aufgabe der Erfindung war daher die Entwicklung eines flüssigen Präparates zur Förderung der Wundheilung bei Menschen und Tieren, vor allem bei Reptilien.

In US 3,608,070 sind filmformende Wundgele bestehend aus Copolymeren von Vinylpyrrolidon und Vinylacetat, einem thixotropen Mittel und Triglyceriden hydroxylierter Fettsäuren beschrieben, die zudem pharmakologisch wirksame Hilfsstoffe enthalten können.

In der Kosmetik und Humanmedizin sind weiterhin ähnliche Prinzipien bekannt. So werden Polyvinylpyrrolidon/vinylacetat-Copolymere (PVP/VA) bereits als kaltwasserbeständige, warmwasserdispergierbare Filmbildner in der Humanmedizin für antiseptische, antibiotische Sprühverbände benutzt. Die eingesetzten PVP/VA-Varianten besitzen Vinylpyrrolidon/Vinylacetat-Mengenvernältnisse zwischen 60/40 und 30/70.

Als desinfizierende Komponenten der neuartigen, filmbildenden Wundgele sollten breitbandig wirkende Desinfizienzien eingesetzt werden, bevorzugt quaternäre Ammoniumsalze.

Überraschend wurden dabei ein neues filmbildendes und desinfizierendes Wundbehandlungsmittel gefunden, das im Stand der Technik bisher noch nicht beschrieben wurde. Auch die Anwendung an Reptilien ist neuartig.

Gegenstand der Erfindung ist daher ein filmbildendes Wundgel mit desinfizierender Wirkung zur Forderung der Wundheilung, insbesondere bei Reptilien, mittels gepufferter PVP/VA-Kombinationsverbindungen mit quaternären Ammoniumsalzen.

Quaternäre Ammoniumsalze, z. B. Benzalkoniumchlorid werden in der Human- und Veterinärmedizin schon seit langer Zeit zur Hautdesinfektion, als Bestandteil von Hautwundreinigern, als Hautdesinfektionsmittel u. v. a. eingesetzt.

Die minimalen mikrobiziden Konzentrationen liegen bei pH 7 im Bereich 50 - 200 mg/l (für Benzalkoniumchlorid) und bei 100 - 400 mg/l bei anderen Quats, z. B. Bradophen [Benzyldodecyl-bis(2-hydroxyäthyl)-ammoniumchlorid]. Bei tieferen pH-Werten (z. B. < 6) verdoppeln bis verzehnfachen sich die erforderlichen minimalen mikrobiziden Konzentrationen, wahrend bei pH-Werten über 7 nur noch ca. 66 % bis 50 % der bei pH 7 erforderlichen Konzentrationen notwendig sind.

Präparate nach dem Stand der Technik stellen bislang nicht sicher, daß die Wirkung im pH-Bereich > 7 erfolgt. Besonders im Hautbereich und in Wunden können pH-Werte von deutlich unter 7 (bis herab zu pH 5) auftreten und dadurch die gewünschte mikrobizide Wirkung in Frage stellen.

Dagegen verhindern in den erfindungsgemäßen Zubereitungen die eingebauten Puffersysteme von pH 7,0 bis 8,0, daß der wirkungsgünstige pH von > 7 unterschritten wird und beugen somit pH-abhängigen Wirkungsverlusten von Quats bei der äußerlichen Anwendung vor.

Zwar sind aus der Humanmedizin Lösungen zur Oberflächendesinfektion bekannt, die pH-erhöhende Komponenten aufweisen, bzw. durch beigefügte Basen (z. B. Amine) auf höhere pH-Werte (ca. pH 9) einstellen können. Solche Zubereitungen können auf der Haut und im Wundbereich auf Grund der sehr hohen pH-Werte zu pH-bedingten Reizungen führen.

Die erfindungsgemäße Lösung beinhaltet einen auf den pH-Bereich 7,0 - 8,0 eingestellten Puffer, insbesondere ein sogenanntes "Tris"/Salzsäure-Puffer-Gemisch ("Tris" = Tris-hydroxymethyl-aminomethan).

Die Kombination eines filmbildenden Wundgels mit breitbandigem Disinfizienz, speziell aus der Gruppe der quaternären Ammoniumsalze und einem auf pH 7,0 - 8,0 eingestellten Puffer - hier speziell ein "Tris"/Salzsäure-Puffer - ist bislang noch nicht bekannt.

Als Lösungsmittel können im Prinzip alle polaren Lösungsmittel, z.B. Wasser oder Alkohole, aber auch weniger polare, nichtprotische Lösungsmittel, wie z. B. Ethylacetat, eingesetzt werden. Ebenso sind Lösungsmittelgemische, wie z. B. aus Isopropanol und Wasser oder aus Isopropanol und Ethylacetat, geeignet.

Für den Einsatz als filmbildendes Wundgel auf PVP/VA, z.B. (60/40)-Basis hat sich ein Gemisch aus Isopropanol und wenig Wasser sehr bewährt. Die Konzentrationen an PVP/VA (60/40) lagen im Bereich 5 - 30 %, vorzugsweise bei 15 - 25 %.

Statt des eingesetzten PVP/VA-Typs der Zusammensetzung PV/VA = 60/40 können auch andere PVP/VA-Typen, z. B. 50/50 oder 30/70 verwendet werden. Die genannten Mischungsverhältnisse sind jeweils gewichtsbezogen.

Die quaternären Ammoniumsalze ("Quats") werden dem Gel in Konzentrationen zwischen 0,05 und 5,0 %, vorzugsweise zwischen 0,1 und 1,0 % zugesetzt. In den untersuchten Rezepturen wurden Benzalkoniumchlorid und vorzugsweise Bradophen [Benzyl-dodecyl-bis(2-hydroryäthyl)ammoniumchlorid] eingesetzt. Ähnlich günstige Wirkung werden auch mit anderen Quats erreicht werden, wenn sie das entsprechende Wirkungsspektrum aufweisen und für den Zweck der Wundheilung geeignet sind.

Als Puffer wurde das System Tris-hydroxymethylaminomethan/Salzsäure ausgewählt. Es sind jedoch auch andere medizinisch und physiologisch geeignete Puffersysteme (hauptsächlich die sogenannten "physiologischen" Puffer) einsetzbar, wenn ihr pks-Wert über 6 - 7 liegt.

Die Gesamtkonzentration des Puffersystems (Base + Salzsäure) lag bei 0,1 % bis 10 %, vorzugsweise bei 0,5 % bis 1,0 %.

Typische Varianten der erfindungsgemäßen, filmbildenden Wundgele werden durch die folgenden Rezepturen beschrieben (Angaben jeweils in Gewichtsprozenten):

### Beispiel 1:

- 73,30 %: Isopropanol
- 2,97 %: Aqua demin.
- 21,00 %: PVP/VA (60/40)
- 0,50 %: "Tris" (Base)
- 2,03 %: 1 M-HCl
- 0,20 %: Bradophen

### Beispiel 2 (mit erhöhter Pufferkapazität):

- 73,30 %: Isopropanol
- 2,97 %: Aqua demin.
- 21,00 %: PVP/VA (60/40)
- 1,00 %: "Tris" (Base)
- 1,53 %: 1 M-HCl
- 0,20 %: Bradophen

Die Lösungen werden nach folgender Vorschrift hergestellt:

Man legt die gesamte Menge Isopropanol vor, verdünnt mit dem Aqua demin. und löst zuerst die TRIS-Base unter Rühren und anschließend die 1molare HCl. Unter kräftigem Rühren wird bei Raumtemperatur das PVP/VA in kleinen Portionen eingetragen und völlig gelost. Zum Schluß setzt man das Bradophen zu und rührt bei Raumtemperatur bis zur vollständigen Auflosung.

In die erfindungsgemäße Zubereitung lassen sich zusätzlich noch weitere, dem Fachmann bekannte Rezepturkomponenten, wie z. B. färbende Stoffe, Farbstoffe und Vitamine, insbesondere der B-Gruppe, einfügen, um das Aussehen zu verändern oder noch zusätzliche Funktionen (z.B. weitere Unterstützung der Wundheilung durch B-Vitamine) zu ermöglichen.

Die erfindungsgemäßen, desinfizierenden Wundgele werden bei Reptilien auf die verletzten oder zu versorgenden Hautpartien in dünner Schicht aufgebracht. Folgende Applikationshilfen lassen sich dabei einsetzen:

Tropfflaschen (Flaschen mit Tropfeinsatz oder Pipetteneinsätzen), mit deren Hilfe das viskose, aber auf den Wunden sehr gut verlaufende Wundgel direkt in der erforderlichen Menge appliziert wird; Wattestabchen oder sogenannte "Q-Tips"; Sprühflaschen (drucklos mit Pumpzerstäuber oder mit Treibmittel).

Etwa 15 - 20 Minuten nach Auftragen der flüssigen Wundgele, wie sie in den Beispielen beschrieben wurden, bildet sich auf der Wunde bzw. der behandelten Hautregion ein elastischer, griffester, nicht mehr klebender Film aus. Dieser Film ist in kaltem Wasser weniger rasch löslich, kann aber mit warmem Wasser nach kurzzeitiger Quellung (über ca. 1 - 5 Min.) leicht und vollständig wieder abgewaschen werden. Dadurch ist die wiederholte Anwendung von frischem, unverbrauchtem Wundgel, z. B. täglich oder zweimal täglich, ohne Probleme möglich.

In bisher allen Fällen konnten bei Reptilien gute Heilungserfolge erzielt werden. Bei der Anwendung der Wundgels im Rahmen einer klinischen Untersuchung konnten bei verschiedenen Reptilienarten und unterschiedlichen Wunden gute Abheilungserfolge erzielt werden. Einzelergebnisse sind auf der nachfolgenden Tabelle I zusammengefaßt:

| Tierart | Art der Wunde | Heilung |
|---|---|---|
| Taggecko (Phelsuma spp) | Bißverletzung am Unterbauch und Oberschenkelinnenseite | gut |
| Boa constrictor | Verbrennung an Unterseite durch Heizmatte | gut |
| Rotschwanzboa | Probenentnahme am Unterkiefer | gut |
| Anolis | Probenentnahme aus Haut (Gichtknoten) | gut |

In zahlreichen Anwendungsversuchen an Säugetieren (Heimtieren, z. B. Hund, Katze) und beim Menschen waren ebenfalls gute bis sehr gute Heilungserfolge bei kleineren Hautverletzungen, Abschürfungen, Schnitten und Stichen, entzündeten Hautregionen, "Pickeln" feststellbar.

Das erfindungsgemäße, filmbildende und desinfizierende Wundgel kann daher auch in den Bereichen Veterinärmedizin, d.h. bei Heim- und Nutztieren (meist Säugetieren, aber auch Geflügel und Ziervögel), Humanmedizin, d.h. beim Menschen mit gutem Erfolg eingesetzt werden.

In die aufgeführte Rezeptur lassen sich auch noch weitere Hilfs- und Wirkstoffe integrieren, z.B. Farbstoffe zur Einfärbung; pharmakologisch wirksame Farbstoffe, z. B. Acridine, Triphenylmethan-Derivate, Thionin-Derivate, Porphyrin-Derivate; antibakteriell wirkende Chemotherapeutika und/oder Antibiotika; Fungizide; antiparasitär wirkende Substanzen; Insektizide, Pestizide (z. B. zur zusätzlichen Behandlung parasitierender Hautinsekten, wie z. B. Milben, Zecken, Flöhe usw.); Vitamine; Carotinoide.

Wie bereits beschrieben, ist die neue Verbindung in polaren und unpolaren Medien löslich und damit applizierbar, z. B. in Alkoholen (z. B. Isopropanol), Wasser, Estern (z. B. Ethylacetat) Lösungsmittelgemischen wie Isopropanol/Wasser, Isopropanol/Ethylacetat.

## Patentansprüche

1. Filmbildendes Wundgel mit desinfizierender Wirkung zur Förderung der Wundheilung aus Polyvinylpyrrolidon/vinylacetat-Copolymeren in Kombination mit quaternären Ammoniumsalzen, dadurch gekennzeichnet, daß es mit einem Puffer auf den pH-Bereich 7,0 - 8,0 eingestellt ist.

2. Filmbildendes Wundgel gemäß Anspruch 1, dadurch gekennzeichnet, daß der Puffer ein Tris-hydroxymethylaminomethan/Salzsäure-Puffer-Gemisch ist.

3. Verfahren zur Herstellung eines Wundgels gemäß der Ansprüche 1 oder 2, dadurch gekennzeichnet, daß man das Lösungsmittel mit Wasser verdünnt, die Puffer-Base einrührt, mit der Puffer-Saure den pH-Wert einstellt, PVP/VA in kleinen Portionen einrührt und anschließend das quaternäre Ammoniumsalz zusetzt.

4. Verwendung von Polyvinylpyrrolidon-/vinylacetat-Copolymeren in Kombination mit quaternären Ammoniumsalzen und einem Puffer gemäß der Ansprüche 1 oder 2 zur Herstellung filmbildender Wundgele mit desinfizierender Wirkung zur Förderung der Wundheilung bei Reptilien, Säugetieren oder Menschen.

## Claims

1. Film-forming wound gel with disinfecting action for the promotion of wound healing from polyvinylpyrrolidone/vinyl acetate co-polymers in combination with quaternary ammonium salts, characterized in that it is adjusted with a buffer to the pH range from 7.0 to 8.0.

2. Wound gel according to claim 1, characterized in that the buffer is a tris-hydroxymethylaminomethane hydrochloric acid buffer mixture.

3. Process for the preparation of a wound gel according claims 1 or 2, characterized in that the solvent used is diluted with water, the buffer base is stirred in, the pH value is adjusted with the buffer acid, PVP/VA is stirred in in small portions and subsequently the quaternary ammonium salt is added thereto.

4. Use of polyvinylpyrrolidone/vinyl acetate copolymers in combination with quaternary ammonium salts and a buffer according to claims 1 or 2 for the manufacturing of film forming wound gels with disinfecting action for the promotion of wound healing on reptiles, mammals and humans.

## Revendications

1. Gel pour blessure formant un film avec effet désinfectant pour favoriser la guérison des plaies, en polyvinylpyrrolidon-vinylacetate-copolymères, en combinaison avec des sels d'ammonium quaternaires, caractérisé par le fait qu'il est réglé selon un tampon dans la zone d'un pH 7,0 - 8,0.

2. Gel pour blessure formant un film conformément à l'revendication 1, caractérisé par le fait que le tampon est un mélange tris-hydroxymethylaminomethan/acide chlorhydrique.

3. Le procédé de fabrication d'un gel pour blessure selon les revendications 1 ou 2, est caractérisé par le fait que l'on délaye le dissolvant avec de l'eau, qu'on le mélange au tampon de base, qu'on fixe le pH de base avec l'acide du tampon, qu'on y mélange PVP/VA en petites portions et rajoute ensuite le sel d'ammonium quaternaire.

4. Utilisation de polyvinylpyrrolidon-/vinylacetate-copolymères en combinaison avec des sels d'ammonium quaternaires et un tampon conformément aux revendications 1 ou 2 pour la fabrication de gels pour les blessures avec effet désinfectant, pour favoriser la guérison de blessures chez des reptiles, mammifères ou êtres humains.
